(19) Europäisches Patentamt
European Patent Office
Office européen des brevets

(11) Publication number: 0 184 169
A2

(12) # EUROPEAN PATENT APPLICATION

(21) Application number: 85115242.1

(22) Date of filing: 30.11.85

(51) Int. Cl.⁴: C 12 N 15/00
C 12 P 21/02

(30) Priority: 04.12.84 JP 257052/84

(43) Date of publication of application:
11.06.86 Bulletin 86/24

(84) Designated Contracting States:
BE DE FR GB NL SE

(71) Applicant: THE GREEN CROSS CORPORATION
15-1, Imabashi-1-chome Higashi-ku Osaka-shi
Osaka 541(JP)

(72) Inventor: Ishida, Yutaka
24-9, Kitanakaburi 1-chome
Hirakata-shi Osaka(JP)

(72) Inventor: Nagai, Masanori
10-10, Nishiyamawaki
Yawata-shi Kyoto(JP)

(72) Inventor: Arimura, Hirofumi
18-1-401, Uenosaka 2-chome
Toyonaka-shi Osaka(JP)

(72) Inventor: Suyama, Tadakazu
3-7, Matsuigaoka 4-chome
Tanabe-cho Tsuzuki-gun Kyoto(JP)

(74) Representative: von Kreisler, Alek, Dipl.-Chem. et al,
Deichmannhaus am Hauptbahnhof
D-5000 Köln 1(DE)

(54) **Method of producing heterologous proteins.**

(57) A method of producing heterologous proteins comprises causing secretion of a heterologous protein into the periplasmic space by using, as the gene expression elements, a expression elements of a gram-positive bacterial strain and, as the host, a gram-negative bacterial strain.

The invention has thus enabled production of heterologous proteins in gram-negative bacteria through the use of gene expression elements of a gram-positive bacterial strain and has contributed to technology diversification in the field of genetic engineering. On the occasion of the secretion of the heterologous protein into the periplasmic space of gram-negative bacteria, the signal peptide is cleaved off, whereby the protein is obtained in the form of a mature protein free of methionine at the N terminal.

EP 0 184 169 A2

# METHOD OF PRODUCING HETEROLOGOUS PROTEINS

## FIELD OF THE INVENTION

This invention relates to a method of producing heterologous proteins by a genetic engineering technique.

## BACKGROUND OF THE INVENTION

Genetic engineering techniques are widely used, for example in the production of useful heterologous proteins.

Escherichia coli, Bacillus subtilis and yeasts, among others, are the hosts which current genetic engineering techniques mainly use. Among them, Escherichia coli may be used most easily as the host because of the most advanced elucidation of the genes in its expression system and so forth. However, the use of Escherichia coli does not always result in efficient recovery of heterologous proteins since they remain in cells after production thereof and are exposed to the decomposing and other actions of intracellular enzymes. On the other hand, the use of Bacillus subtilis as the host might result in more efficient recovery of heterologous proteins produced since they are secrected out of cells. However, the expression system in Bacillus subtilis remains unknown in may respects, so that it is not yet always suitable to use Bacillus subtilis in commercial production of heterologous proteins.

An object of the invention is to establish a system which is capable of causing secretion of heterologous proteins into the periplasmic space using gram-negative bacteria.

## SUMMARY OF THE INVENTION

The invention provides a genetic engineering technique by which heterologous proteins are secreted into the periplasmic space using, as the gene expression elements, expression elements of a gram-positive bacterial strain and, as the host, a gram-negative bacterial strain.

## BRIEF DESCRIPTION OF THE DRAWINGS

In the accompaning drawings,

Fig. 1 shows the restriction enzyme map of pGC303,

Fig. 2 shows the scheme of cloning the $\alpha$-amylase gene from Bacillus natto,

Fig. 3 shows the process for constructing an expression vector for use in Escherichia coli, the numerical figures indicating the respective steps referred to in the detailed description of the invention,

Fig. 4 shows the process for constructing pI$\alpha_2$amy$_2$ by ligation of pY16-1 with the IFN-$\alpha_2$ gene,

Fig. 5 shows the process for constructing $pI\alpha_2trap31$, which carries the IFN-$\alpha_2$ gene, from 
$pI_2trp1$ (Japanese Patent Application No. 250988/1984), and

Fig. 6 shows the process for preparation of $pI\alpha_2$ trp1,
Fig. 7 shows the process for preparation of mature IFN-$\alpha_2$,
Fig. 8 shows the restriction cleavage map of DNA fragment (883 bp),

indicating the region coding for mature IFN-$\alpha_2$,

indicating the region non-coding for IFN-$\alpha_2$,

indicating the trp promoter region,

indicating the trp terminator region,

RI indicating EcoRI and HIII indicating HindIII.

$Tc^r$ : Tetracycline resistance

$CM^r$ : Chloramphenicol resistance

$SM^r$ : Streptomycin resistance

$Ap^r$ : Ampicillin resistance

amy : Gene coding for $\alpha$-amylase

BAP : Bacterial alkaline phosphatase

Ori : Replication origin

P/O : Promoter-operator region

trp P/O : Tryptophan promoter-operator region

## DETAILED DESCRIPTION OF THE INVENTION

(a) Preparation of gene expression elements

The gene expression elements to be used in accordance with the invention is of the gram-positive bacterial strain. The gram-positive bacterial strain is preferably a strain belonging to the genus Bacillus, such as a strain of Bacillus subtilis or Bacillus natto. The term gene expression elements" includes within the meaning thereof the promoter, SD sequence, signal sequence coding region and so on which are of the gram-positive bacterial strain origin. A preferred example of the gene expression elements is of the $\alpha$-amylase gene. The $\alpha$-amylase gene has been isolated by colleagues to the present inventors from Bacillus natto GC161 deposited at the Fermentation Research Institute under the deposit number FERM BP-539 (cf. European Patent Publication No. 135045; U.S. Ser. 633562). A partial nucleotide sequence containing the $\alpha$-amylase promoter and signal sequence coding region is shown in Table 1.

Tabel 1

GTAC AGTCTCGGGC

-200

AGTTTTTTTT ATAGGAACAT TGATTTGTAT TCACTCTGCC AAGTTGTTTT

-150

GATAGAGTGA TTGTGATAAT TTAAAATGTA AGCGTTAACA AAATTCTCCA

-100

GTCTTCACAT CAGCTTGAAA GGAGGAAGCG GAAGAATGAA GTAAGAGGGA

-50 -1

TTTTTGACTC CGAAGTAAGT CTTCAAAAAA TCAAATAAGG AGTGTCAAGA

1 50

ATGTTTGCAA AACGATTCAA AACCTCTTTA CTGCCGTTAT TCGCTGGATT

└→ $\alpha$-amylase 100

TTTATTGCTG TTTTATTTGG TTCTGGCAGG ACCGGCGGCT GCGAGTGCTG

150

AAACGGCGAA CAAATCGAAT GAGCTTACAG CACCGTCGAT CAAAAGCGGA

200

ACCATTCTTC ATGCATGGAA TTGGTCGTTC AATACGTTAA AACACAATAT

250

GAAGGATATT CATGATGCAG GATATACAGC CATTCAGACA TCTCCGATTA

300

ACCAAGTAAA GGAAGGGAAT CAAGGAGATA AAAGCATGTC GAACTGGTAC

A plasmid containing this gene expression elements is provided under the code pGC303 which is a derivative of the plasmid pGC200 derived from a strain of Bacillus natto deposited at the Fermentation Research Institute under the deposit number FERM BP-645(cf. European Patent Publication No. 135045; U.S. Ser. 633562). The restriction enzyme map of pGC303 is shown in Fig. 1.

(b) Preparation of expression vector for use in gram-negative bacteria

The gene expression elements prepared in step (a) is then inserted into such a plasmid as pBR322 derived from Escherichia coli, a gram-negative bacterial species, to creat a plasmid capable of causing expression of heterologous proteins in gram-negative bacteria by the action of the gram-positive bacterial gene expression elements.

(c) Ligation with heterologous protein gene

A heterologous protein gene coding for a physiologically active substance of significant use is ligated with the expression vector in gram-negative bacteria as obtained in step (b), at a site downstream from the promoter, SD sequence and signal sequence of said vector, by the known restriction enzyme and ligase treatment process. The ligation product is introduced into an appropriate host. Preferred exmples of the physiologically active substance are interferons, urokinase, TPA, IL-2 and HBs antigen.

(d) Transformation

As the host preferred is a gram-negative bacterial strain, in particular a strain of *Escherichia coli*. The host is transformed by the known method [Cohen, S.N. et al., Proc. Natl. Acad. Sci. USA, 69, 2110 (1972)], for instance.

(e) Cultivation of host

The transformant obtained in step (d) is cultivated on a *per se* known medium suited for growing the host. A preferred example of the medium is the medium used by Palva et al. for growing *Bacillus subtilis* [Gene, 22, 229 (1983)] or a modification thereof. The cultivation is generally conducted at 15-43 ℃ for 3-24 hours, with aeration and/or stirring as necessary.

(f) Recovery of heterologous protein

After cultivation, cells are harvested by a known method (e.g. centrifugation) and the periplasmic fraction is recovered by a known method for recovering proteins secreted into the periplasm (e.g. osmotic shock method (Nossal, N.G. and Heppel, L.A., J. Biol. Chem., 241, 3055 (1966)) ) or a modification thereof. The protein is recovered from this fraction by a *per se* known method, for example by affinity chromatography and/or fractionation based on solubility differences.

The invention has thus enabled production of heterologous proteins in gram-negative bacteria through the use of gene expression elements from gram-positive bacterial strains and has contributed to technology diversification in the field of genetic engineering. On the occasion of the secretion of the heterologous protein into the periplasmic space of gram-negative bacteria, the signal peptide is cleaved off, whereby the protein is obtained in the form of a mature protein free of methionine at the N terminal. Thus, the invention can provide an efficient method of producing heterologous proteins using genetic engineering technology.

In the following, the process for producing human interferon-$\alpha_2$ (hereinafter, IFN-$\alpha_2$) as a preferred example of the heterologous protein using $\alpha$-amylase promoter of the *Bacillus natto* as a preferred example of gene expression elements of gram-positive bacterial strain and *Escherichia coli* as a preferred exmaple of the host will be described step by step. It is to be noted, however, this mode of practice is by no means limitative of the scope of the present invention.

(a) Cloning of gene expression elements

A one-liter portion of a overnight culture of Bacillus natto GC161 (deposited at the Fermentation Research Institute under the deposit number FERM BP-539) was centrifuged and the cells obtained were washed. To the cells were added 10 ml of 20 % sucrose-added 50 mM Tris-hydrochloride buffer (pH 8.0) and 100 mg of lysozyme, followed by reaction at 37°C for 15 minutes. Thereafter, 10 ml of 1% Sarkosyl-1% EDTA solution and 1 ml of Pronase E (100 mg/ml) were added. After standing at 37°C for 16 hours, the solution was made up to 35 ml with TES solution (30 mM Tris-HCl, 50 mM NaCl, 5 mM EDTA, pH 8). Thereto were added 35 g of cesium chloride and 1 ml of ethidium bromide (10 mg/ml) and the mixture was centrifuged at 60,000 rpm (Beckman 70.1 Ti rotor) for 16 hours. The chromosomal DNA band was collected, dialyzed against TES solution and treated twice with phenol for purification of the chromosomal DNA.

The chromosomal DNA thus obtained was cleaved with the restriction enzymes PvuII, BglII, HpaI, BamHI, HindIII and so forth. Fragments were extracted from 0.8% agarose gel and ligated with various plasmids, pC194, pE194, pUB110, and pGC200 (deposited at the Fermentation Research Institute under the deposit number FERM BP-645) which is a derivative of pC194, and the ligation products were used for transforming B. subtilis ($rec^-$, $amy^-$). After selection based on drug resistance of each plasmid, the transformants selected were inoculated onto starch-added agar plates (L-agar-1% soluble starch). After incubation at 37 °C for 2 days, iodine solution (0.1% iodine-1% KI) was added to the agar plates for checking α-amylase activity on the basis of decoloration of iodine. When, among the five restriction enzymes mentioned above, HindIII was used for cleaving the chromosomal DNA prepared from Bacillus natto and a fragment of said DNA was inserted into pGC200 at the HindIII site thereof, followed by transformation with the resulting plasmid, there was obtained a strain having α-amylase activity. The chromosomal DNA fragment inserted was a 7 kb fragment. Therefore, the 7 kb DNA containing the α-amylase gene was excised using the restriction enzymes HindIII and RsaI and inserted into the EcoRV-HindIII fragment of pBR322. The plasmid pGC302 thus obtained was then cleaved with SacII, followed by ligation, which gave pGC303 (7.9 kb) (Fig. 2). Transformation was performed in the conventional manner, selection was made based on the ampicillin resistance and the transformants selected were checked for α-amylase activity in the same manner as above, whereupon expression of α-amylase was confirmed. Thus was confirmed the presence of the whole α-amylase gene series. In this manner, the plasmid pGC303 containing the α-amylase promoter, signal sequence coding region and structural gene for α-amylase was obtained.

The DNA sequence of the promoter and signal sequence coding region of α-amylase gene was determined by the Maxam-Gilbert method (Maxam, A. and Gilbert, W., 1979, Methods in Enzymology, 65, 499-560) (Table 1).

(b) Construction of expression vector for use in <u>Escherichia</u> <u>coli</u>

An expression vector for use in <u>Escherichia</u> <u>coli</u> was constructed from pGC303 and pBR322 in accordance with the procedure shown in Fig. 3.

Step 1: pGC303 was digested with the restriction enzymes <u>Ban</u>III and <u>Ban</u>II to give a relatively large DNA fragment containing the promoter and signal sequence coding region of α-amylase gene. The reaction was carried out using the following reaction mixture composition:

| | | |
|---|---|---|
| pGC303 (1.0 μg/μℓ) | 100 | μℓ |
| 10×<u>Bcl</u>I buffer *1 | 20 | μℓ |
| <u>Ban</u>II (8 U/μℓ, Toyobo) | 20 | μℓ |
| <u>Ban</u>III (5 U/μℓ, Toyobo) | 20 | μℓ |
| $H_2O$ | 40 | μℓ |
| Total | 200 | μℓ |

*1 ┌ 60 mM Tris-HCl (pH 7.4)

100 mM $MgCl_2$
10 mM dithiothreitol
750 mM KCl

The reaction was carried out overnight at 37℃. After completion of the reaction, the whole reaction mixture was applied to 1% agarose gel, followed by electrophoresis. A 960 bp fragment was recovered following adsorption on DEAE-paper. Thereto was added 60μg of tRNA as the carrier for ethanol precipitation. The mixture was extracted with phenol, and the DNA was recovered by ethanol precipitation. The DNA precipitate was dissolved in 90 μℓ of $H_2O$.

Step 2: The fragment obtained in step 1 was partially digested with AluI using the following reaction mixture composition:

| | |
|---|---|
| DNA fragment solution obtained in step 1 | 50 μℓ |
| 10×AluI buffer *2 | 20 μℓ |
| AluI (2 U/μℓ, Takara Shuzo) | 20 μℓ |
| $H_2O$ | 110 μℓ |
| Total | 200 μℓ |

*2
100 mM Tris-HCl (pH 7.5)
70 mM $MgCl_2$
200 mM NaCl
70 mM 2-mercaptoethanol

The reaction was carried out at 37℃ for 5

minutes, and the whole reaction mixture was applied to 5% polyacrylamide gel, followed by electrophoresis. Thereafter, a 621 bp DNA fragment was recovered by electroelution. To the fragment fraction was added 20μg of polyinosic acid was added as the carrier in ethanol precipitation. After extraction with phenol, the fragment was precipitated with ethanol. The DNA precipitate obtained was dissolved in 30 μℓ of $H_2O$.

Step 3: A BamHI linker was ligated with the DNA fragment obtained in step 2 at the AluI end thereof using the following reaction mixture composition:

| | |
|---|---|
| Partial AluI digest fragment obtained in step 2 | 10 μℓ |
| 10×ligation buffer *3 | 1 μℓ |
| BamHI linker *4 | 10 μℓ |
| $T_4$ DNA ligase (175 U/μℓ Takara Shuzo) | 2 μℓ |
| Total | 23 μℓ |

*3
- 660 mM Tris-HCl (pH 7.6)
- 10 mM ATP
- 10 mM spermidine
- 100 mM $MgCl_2$
- 150 mM DTT (dithiothreitol)

*4
- Product of Pharmacia P-L Biochemicals
- Sequence: 5'-d(CCCGGATCCGGG)-3'
- Used after phosphorylation at the 5' end.

The reaction was carried out overnight at 4 °C, followed by extraction with phenol and precipitation with ethanol. The DNA precipitate was dissolved in 42 μℓ of $H_2O$.

Step 4: The DNA fragment obtained in step 3 was digested with BamHI using the following reaction mixture composition:

| | |
|---|---|
| DNA obtained in step 3 | 42 μℓ |
| 10×BamHI buffer *5 | 5 μℓ |
| BamHI (12 U/μℓ, Takara Shuzo) | 3 μℓ |
| Total | 50 μℓ |

*5
- 100 mM Tris-HCl (pH 8.0)
- 70 mM $MgCl_2$
- 1 M NaCl
- 20 mM 2-mercaptoethanol

The reaction was carried out overnight at 37°C and the whole reaction mixture was applied to 5% polyacrylamide gel for electrophoresis. After electrophoresis, a 533 bp fragment was recovered by electroelution, followed by extraction with phenol and precipitation with ethanol. The DNA precipitate recovered was dissolved in 50 μℓ of $H_2O$.

Step 5: For use as a vector for cloning the DNA fragment (both ends being BamHI ends) obtained in step 4, pBR322 was digested with BamHI overnight at 37 °C using the following reaction mixture composition:

| | | |
|---|---|---|
| pBR322 (1.0 μg/μℓ) | | 25 μℓ |
| 10×*Bam*HI buffer(mentioned above) | | 5 μℓ |
| *Bam*HI (10 U/μℓ, Takara Shuzo) | | 3 μℓ |
| $H_2O$ | | 17 μℓ |
| | Total | 50 μℓ |

and then the phosphoric acid residue at the 5' end was eliminated by using bacterial alkaline phosphatase(BAP).

After reaction, DNA was recovered by precipitation with ethanol. The DNA was dissolved in 180 μℓ of $H_2O$.

To prevent self ligation of the vector (pBR322), this was treated with bacterial alkaline phosphatase (BAP) at 65 ℃ for 40 minutes for eliminating the phosphoric acid residue at the 5' end. The reaction mixture composition was as follows:

| | | |
|---|---|---|
| *Bam*HI-digested pBR322 | | 180 μℓ |
| 10×BAP buffer *6 | | 20 μℓ |
| BAP (0.45 U/μℓ, Takara Shuzo) | | 2 μℓ |
| | Total | 202 μℓ |

*6
- 100 mM Tris-HCl (pH 7.5)
- 70 mM $MgCl_2$
- 600 mM NaCl

After reaction, phenol extraction was repeated three times and DNA was recovered by precipitation with ethanol. The DNA precipitate was dissolved in 25 μℓ of $H_2O$.

Step 6: The DNA fragment obtained in step 4 and the vector obtained in step 5 were ligated together by overnight reaction at 4 ℃. The reaction mixture composition was as follows:

| | |
|---|---|
| DNA fragment from step 4 | 10 μℓ |
| BamHI-digested and BAP-treated pBR322 | 5 μℓ |
| 10×ligation buffer *7 | 4 μℓ |
| $T_4$ DNA ligase (175 U/μℓ, Takara Shuzo) | 1 μℓ |
| Total | 20 μℓ |

*7
- 660 mM Tris-HCl(pH 7.6)
- 50 mM $MgCl_2$
- 50 mM DTT(dithiothreitol)
- 10 mM ATP

After reaction, the whole reaction mixture was used for transformation of E. coli RR1 by the method of Cohen et al. As a result, there were obtained 6,500 colonies, 200 of which were checked for sensitivity to ampicillin (Ap) and to tetracycline (Tc). Fourteen strains were found to be resistant to Ap ($Ap^r$) and susceptible to Tc ($Tc^s$). Using these 14 strains, the plasmids were prepared by the alkaline extraction method (Birnboim, H. C. and Doly, J., Nucleic Acids Research, 7, 1513 (1979)), the plasmids were then examined for their structure using varous restriction enzymes. As a result, 2 colonies were found to contain a plasmid having the structure of pYI5-1 given in Fig. 3. One of these strains was used for large-quantity plasmid preparation and the plasmid was named pYI5-1.

Step 7: pYI5-1 is inconvenient for inserting heterologous genes such as the IFN-$\alpha_2$ gene since it has two BamHI sites. Therefore, for causing one BamHI site (upstream from the $\alpha$-amylase promoter) to disappesr, pYI5-1 was first digested with AvaI at 37℃ overnight. The reaction mixture composition used was as follows:

| | | |
|---|---|---|
| pYI5-1 (1.43 μg/μℓ) | | 14 μℓ (20 μg) |
| 10×AvaI buffer *8 | | 5 μℓ |
| AvaI (8 U/μℓ, Takara shuzo) | | 3 μℓ |
| $H_2O$ | | 28 μℓ |
| | Total | 50 μℓ |

*8
- 100 mM Tris-HCl (pH 8.0)
- 70 mM $MgCl_2$
- 600 mM NaCl
- 70 mM 2-mercaptoethanol

After reaction, the whole reaction mixture was applied to 1% agarose gel and electrophoresis was performed. A DNA fragment of about 3.5 kbp was caused to be adsorbed on DEAE-paper and then recovered. As the carrier to serve in precipitation with ethanol, there was added 20 μg of tRNA. Following extraction with phenol, DNA was recovered by precipitation with ethanol. The DNA recovered was dissolved in 50 μl of $H_2O$.

Step 8: The AvaI-digested pYI5-1 obtained in step 7 was cyclized by allowing self ligation to take place at 4°C overnight. The reaction mixture composition used was as follows:

| | |
|---|---|
| AvaI-digested pYI5-1 obtained in step 7 | 2 μl |
| 10× ligation buffer (same in composition as in step 6) | 10 μl |
| $T_4$ DNA ligase (175 U/μl, Takara Shuzo) | 1 μl |
| $H_2O$ | 87 μl |
| Total | 100 μl |

A 25 μl portion of the reaction mixture (100 μl) was used for transformation of *E. coli* RR1, whereby 18 ampicillin-resistant ($Ap^r$) colonies were obtained. These colonies were subjected to plasmid extraction by the alkline extraction method and the plasmids extracted were examined for their structure using various restriction enzymes. As a result, 14 colonies were found to have a plasmid having the structure of pYI6-1 given in Fig. 3. One of these strains was used for large-quantity plasmid preparation and the plasmid obtained was named pYI6-1.

The plasmid pYI6-1 thus obtained has, in addition to the BamHI site, one each of EcoRI, ClaI (same as BanIII), HindIII and EcoRV sites. Using these sites, heterologous genes such as the IFN-$\alpha_2$ gene can be ligated to said plasmid. Thus, DNA fragments having a BamHI site upstream from the gene coding for a heterologous protein such as IFN-$\alpha_2$ and having a flush end site such as an EcoRI, ClaI, HindIII or EcoRV site downstream therefrom can be ligated with said plasmid.

(c) Ligation of pYI6-1 and IFN-$\alpha_2$ gene (construction of pI$\alpha_2$amy$_2$)

For effecting expression of the IFN-$\alpha_2$ gene using pYI6-1, a DNA fragment containing the IFN-$\alpha_2$ gene was ligated to pYI6-1 between the BamHI site and the EcoRV site thereof, whereby pI$\alpha_2$amy$_2$ was obtained. This process is summarized in Fig. 4.

Step 1: pYI6-1 was digested with BamHI and EcoRV at 37 ℃ overnight. The reaction mixture composition used was as follows:

| | |
|---|---|
| pYI6-1 (0.508 μg/μℓ) | 100 μℓ |
| 10×BamHI buffer | 15 μℓ |
| BamHI (12 U/μℓ, Takara shuzo) | 10 μℓ |
| EcoRV (5 U/μℓ, Takara Shuzo) | 20 μℓ |
| $H_2O$ | 5 μℓ |
| Total | 150 μℓ |

After reaction, the whole reaction mixture was applied to 1% agarose gel and electrophoresis was conducted. After electrophoresis, a DNA fragment of about 3.4 kbp was allowed to be adsorbed on DEAE-paper.

Following elution of DNA and one extraction with phenol, the DNA was recovered by precipitation with ethanol. The DNA was dissolved in 50 $\mu l$ of $H_2O$.

Step 2: An IFN-$\alpha_2$ gene-bearing plasmid (pI$\alpha_2$-trp31) was prepared from pI$\alpha_2$trp1 (Japanese Patent Application No. 250988/84) following the process given in Fig. 5.

The IFN-$\alpha_2$ gene, with the trp a terminator, was excised by digesting pI$\alpha_2$trp31 with BamHI and SmaI at 37°C overnight. The reaction mixture composition used was as follows:

| | |
|---|---|
| pI$\alpha_2$trp31 (2.22 $\mu g/\mu l$) | 22.5 $\mu l$ (50 $\mu g$) |
| 10×BamHI buffer | 10 $\mu l$ |
| BamHI (12 U/$\mu l$, Takara Shuzo) | 7 $\mu l$ |
| SmaI (8 U/$\mu l$, Takara Shuzo) | 10 $\mu l$ |
| $H_2O$ | 50.5 $\mu l$ |
| Total | 100 $\mu l$ |

After reaction, the whole was applied to 1% agarose gel, followed by electrophoresis. A DNA fragment of about 950 bp was allowed to be adsorbed on DEAE-paper, and then the DNA was recovered in the same manner as above. The DNA was dissolved in 50 $\mu l$ of $H_2O$.

pI$\alpha_2$ trp1 was prepared by the following method (see Fig 6) :

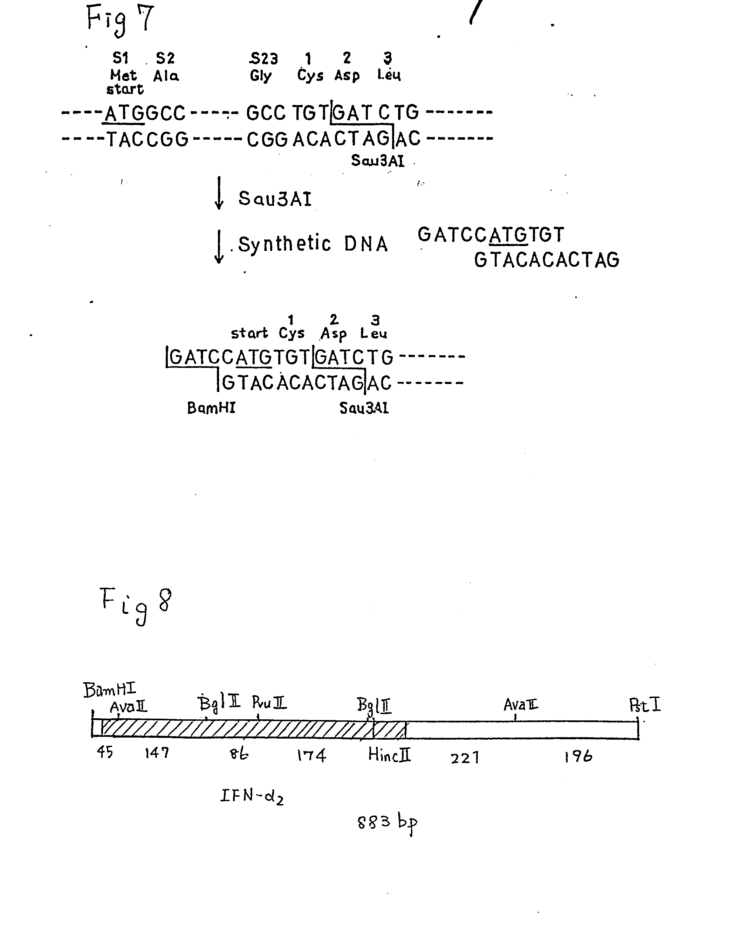

The cDNA fragment coding for IFN-$\alpha_2$ was prepared by a method similar to that of described in European Patent Publication No. 32134, and then ATG-combined matured IFN-$\alpha_2$ gene, which was combined ATG, was prepared by the process given in Fig 7.

The restriction cleavage map of DNA fragment (883 bp) thus obtained was shown in Fig. 8.

The pYN6 (see European Patent Publication No. 152830) was digested with EcoRI, then digested product was treated with Bacterial Alkaline Phosphatase, and then digestion with BamHI was conducted.

The reaction mixture was applied to 6 % polyacrylamide gel (PAGE), followed by electrophoresis, whereby a 69 bp fragment was isolated.

The 69 bp fragment was ligated with IFN-$\alpha_2$ gene-bearing 883 bp, and then a fragment corresponding to 952 bp was isolated by using 5 % PAGE.

Thus obtained fragment was ligated with a EcoRI-PstI fragment (3,610 bp) in pBR322. And E. coli RR1 was transformed by this obtained plasmid.

Thus obtained $Tc^r$ transformants were screened and plasmid were extracted from these transformants. And these plasmid were examined using various restriction enzymes. Acontemplated plasmid pI $\alpha_2$ trpl(4,562 bp)" was obtained.

Step 3: The vector prepared in step 1 and the IFN-$\alpha_2$ gene-containing DNA fragment prepared in step 2 were ligated together by overnight reaction at 12 ℃ using the following reaction mixture composition:

| | |
|---|---|
| BamHI-EcoRV-digested pYI6-1 | 2 μl |
| BamHI-SmaI-digested DNA fragment | 5 μl |
| 10× ligation buffer | 2.5 μl |
| $T_4$ DNA ligase (175 U/μl, Takara Shuzo) | 2 μl |
| $H_2O$ | 13.5 μl |
| Total | 25 μl |

(d) Transformation

After the reaction in the above step 3, a 16 μl portion of the reaction mixture was used for transformation of E. coli HB101. As a result, 9 $Ap^r$ transformants were obtained. These strains were subjected to plasmid extraction by the alkaline method and the plasmids extracted were examined for their structure using various restriction enzymes. All the strains were found to have $pI\alpha_2amy_2$ shown in Fig. 4. One of these strains ($pI\alpha_2amy_2$-carrying E. coli HB101) was checked for its ability to produce IFN-$\alpha_2$.

(e) Production of IFN-$\alpha_2$

The $pI\alpha_2amy_2$-carrying strain of E. coli HB101 was grown in a 2-liter erlenmeyer flask with shaking at 37℃ using 500 ml of a medium (specified below) according to Palva et al. [Gene, 22, 229 (1983)], with checking at timed intervals for bacterial growth in terms of measured $A_{650}$ values.

| | |
|---|---|
| Yeast extract (Difco) | 10 g/liter |
| Bactotryptone (Difco) | 20 g/liter |

| | |
|---|---|
| Soluble starch (Merck) | 10 g/liter |
| NaCl | 10 g/liter |
| $(NH_4)_2SO_4$ | 1 g/liter |
| $K_2HPO_4$ | 7 g/liter |
| $KH_2PO_4$ | 2 g/liter |
| Ampicillin | 25 mg/liter |

(f) Recovery of IFN-$\alpha_2$

The culture obtained in (e) was sampled (20 ml) at 3-hour intervals and the samples were each fractionated according to the flowchart given later herein. The culture supernatant, cell washings (1) and (2), periplasmic fraction and cell extract were assayed for IFN titer by the RPHA method. At 9 hours after start of cultivation, the IFN-$\alpha_2$ potencies thus measured are shown in Table 2.

Table 2

| | IFN-$\alpha_2$ titer (IU/liter of medium) |
|---|---|
| Culture supernatant | $1.88 \times 10^6$ |
| Cell washings | $6.25 \times 10^6$ |
| Cell extract | $2.65 \times 10^6$ |
| Periplasmic fraction | $1.88 \times 10^6$ |

Then, the periplasmic fractions and cell extract fractions collected at 3-hour intervals over the period of hour 6 to hour 27 were assayed for IFN-$\alpha_2$ potency. The results thus obtained are shown in Table 3. As is evident from the data given in Table 3, the yield increased with the growth of bacteria and became constant at 15 hours after start of cultivation. Not less than 95% of intracellular IFN-$\alpha_2$ was found secreted in the periplasm.

Table 3

IFN-$\alpha_2$ titer (IU/liter of medium)

| Cultivation period (hours) | Periplasmic fraction | Cell extract | $A_{650}$ (adsorbance) |
|---|---|---|---|
| 6 | $6.63 \times 10^7$ | $2.65\times10^6$ | 3.748 |
| 9 | $1.88 \times 10^8$ | $2.65\times10^6$ | 7.27 |
| 12 | $2.66 \times 10^8$ | $5.30\times10^6$ | 9.65 |
| 15 | $3.75 \times 10^8$ | $5.30\times10^6$ | 11.03 |
| 18 | $3.75 \times 10^8$ | $5.30\times10^6$ | 11.43 |
| 21 | $3.75 \times 10^8$ | $7.50\times10^6$ | 11.71 |
| 24 | $3.75 \times 10^8$ | $1.50\times10^7$ | 11.13 |
| 27 | $5.32 \times 10^8$ | $3.00\times10^7$ | 10.29 |

Flowchart illustrating the process for recovering IFN-$\alpha_2$

20 ml of culture

↓ Centrifugation (8,000 rpm, 4℃, 10 minutes)

Cell pellet → Supernatant (culture supernatant)

↓ Suspended in 5 ml of cold aqueous solution containing 10 mM Tris-HCl (pH 8.0) and 30 mM NaCl

↓ Centrifugation (6,000 rpm, 4℃, 5 minutes)

Cell pellet → Supernatant (cell washings) (1)

↓ Suspended in 5 ml of aqueous solution containing 20% sucrose and 30 mM Tris-HCl (pH 8.0)

↓ Addition of 10 μl of 0.5 M EDTA (pH 8.0) (final concentration 1 mM EDTA)

↓ Shaking at room temperature for 10 minutes

↓ Centrifugation (6,000 rpm, 4℃, 5 minutes)

(to be continued)

Cell pellet → Supernatant (cell washings) (2)

Cell pellet

Suspended in 5 ml of cold distilled water

Shaking on an ice bath for 10 minutes

Centrifugation (6,000 rpm, 4℃, 5 minutes)

Cell pellet → Supernatant (periplasmic fraction)

Suspended in 1.5 ml of lysis buffer*

Allowed to stand at 0℃ for 30 minutes

Five repetitions of freezing-thawing cycle

Centrifugation (18,000 rpm, 4℃, 90 minutes)

Precipitate → Supernatant (cell extract)

* 50 mM Tris-HCl (pH 8.0), 30 mM NaCl, 6 mM EDTA, 10 μg/ml phenylmethylsulfonyl fluoride, 1 mg/ml lysozyme

What is claimed is:

1. A method of producing a heterologous protein which comprises causing secretion of a heterologous protein into the periplasmic space by using, as the gene expression elements, expression elements of a gram-positive bacterial strain and, as the host, a gram-negative bacterial strain.

2. The method of claim 1, wherein the gram-positive bacterial strain is a strain of the genus Bacillus.

3. The method of claim 1, wherein the gram-negative bacterial strain is a strain of Escherichia coli.

4. the method of claim 1, wherein the heterologous protein is interferon-$\alpha$.

5. The method of claim 1, wherein the gene expression elements contain $\alpha$-amylase promoter portion of Bacillus natto.

FIG 1

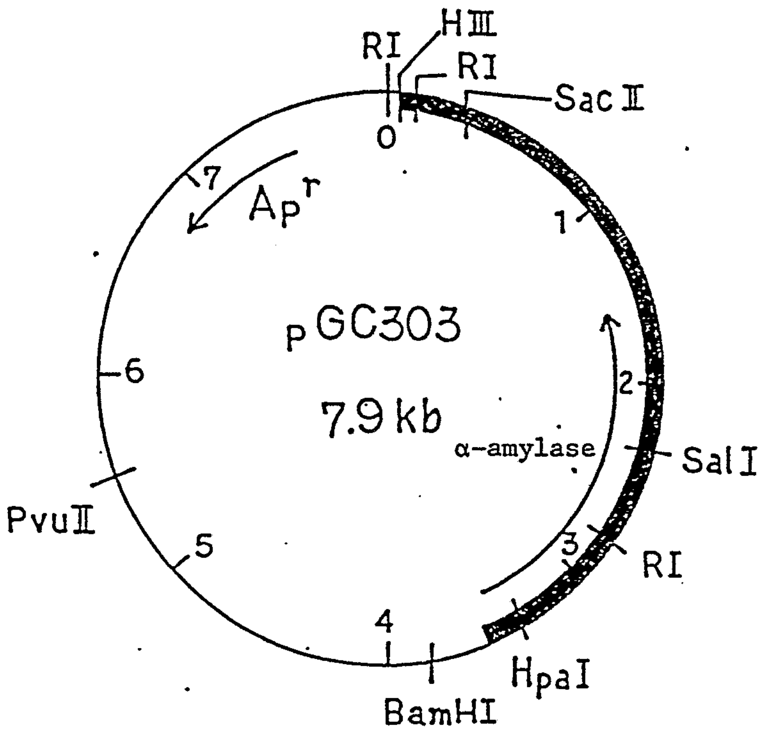
RI
HIII
RI
SacII
0
1
2
3
4
5
6
7
Apr
pGC303
7.9 kb
α-amylase
SalI
RI
HpaI
BamHI
PvuII

FIG 2



0184169

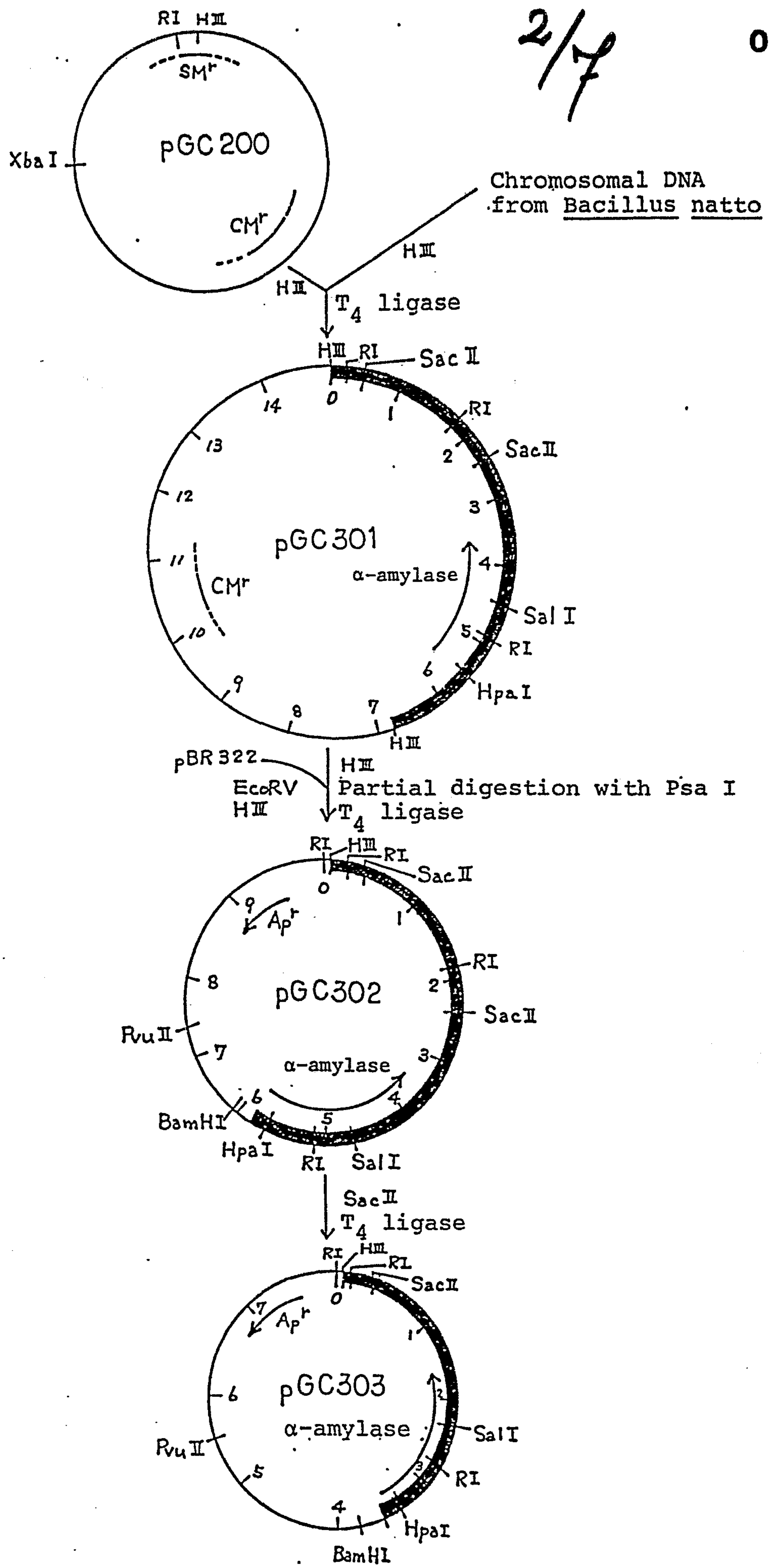

FIG 3

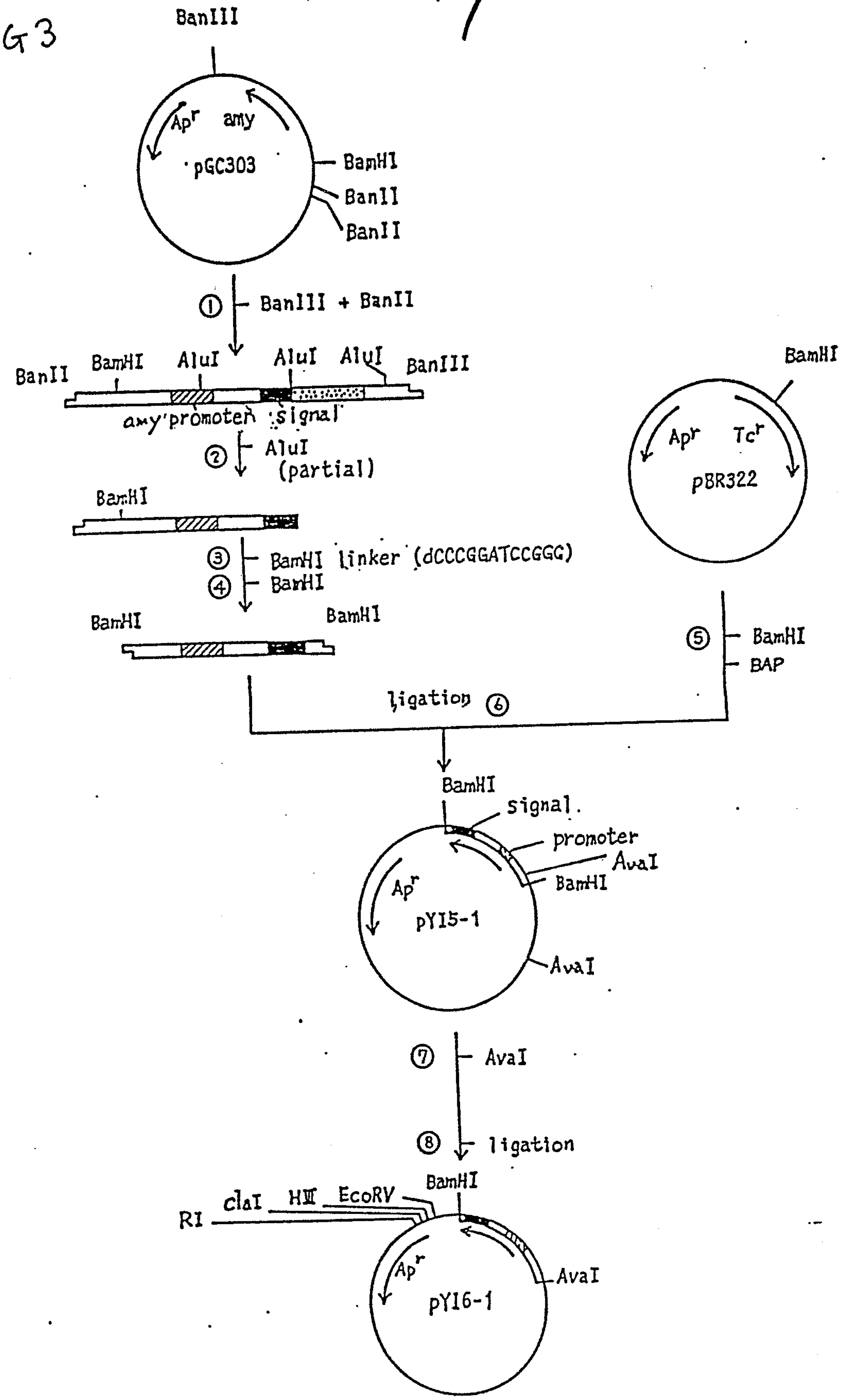
BanIII
Apr
amy
pGC303
BamHI
BanII
BanII
① BanIII + BanII
BanII
BamHI
AluI
AluI
AluI
BanIII
amy promoter
signal
② AluI (partial)
BamHI
③ BamHI linker (dCCCGGATCCGGG)
④ BamHI
BamHI
BamHI
BamHI
Apr
Tcr
pBR322
⑤ BamHI
BAP
ligation ⑥
BamHI
signal.
promoter
AvaI
BamHI
Apr
pYI5-1
AvaI
⑦ AvaI
⑧ ligation
RI
ClaI
HIII
EcoRV
BamHI
Apr
pYI6-1
AvaI

FIG 4

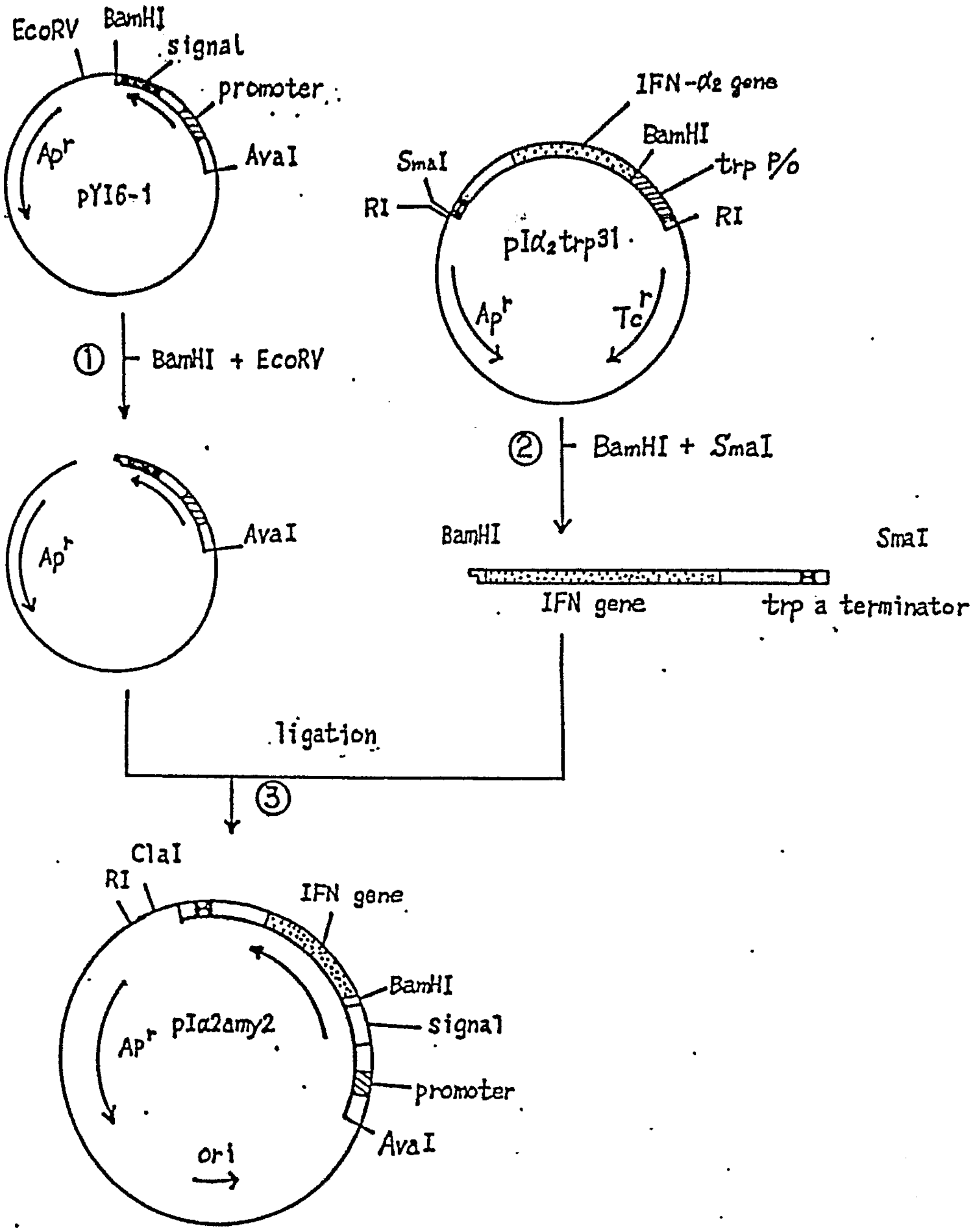
EcoRV
BamHI
signal
promoter
AvaI
Ap^r
pYI6-1
IFN-α2 gene
BamHI
trp P/O
SmaI
RI
RI
pIα2trp31
Ap^r
Tc^r
① BamHI + EcoRV
② BamHI + SmaI
AvaI
Ap^r
BamHI
SmaI
IFN gene
trp a terminator
ligation
③
ClaI
RI
IFN gene
BamHI
signal
promoter
AvaI
Ap^r
pIα2amy2
ori

FIG 5

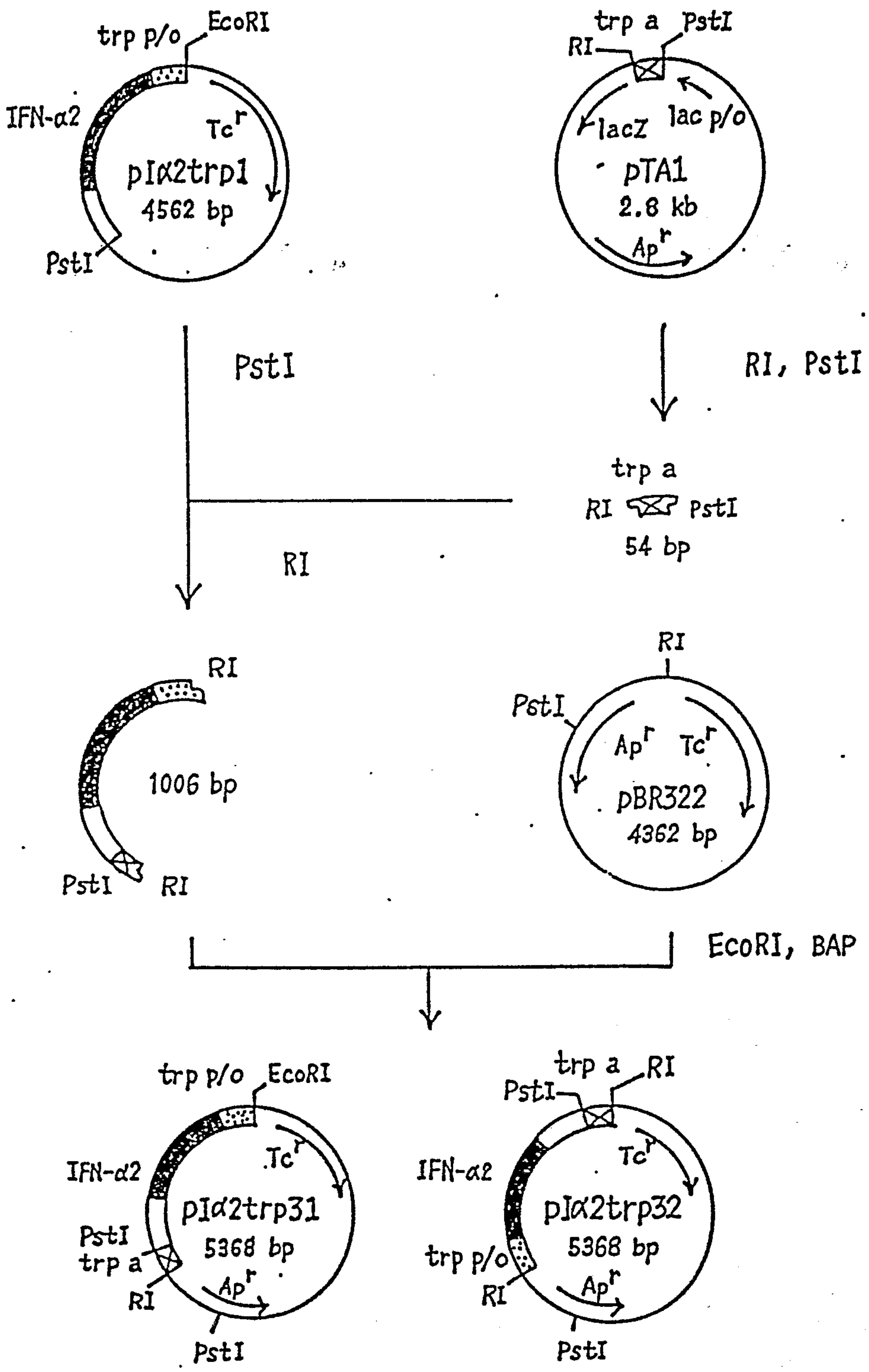
trp p/o
EcoRI
IFN-α2
Tc^r
pIα2trp1
4562 bp
PstI
trp a
RI
PstI
lacZ
lac p/o
pTA1
2.8 kb
Ap^r
PstI
RI, PstI
trp a
RI
PstI
54 bp
RI
RI
1006 bp
PstI
RI
RI
PstI
Ap^r
Tc^r
pBR322
4362 bp
EcoRI, BAP
trp p/o
EcoRI
IFN-α2
Tc^r
pIα2trp31
5368 bp
PstI
trp a
RI
Ap^r
PstI
trp a
RI
PstI
IFN-α2
Tc^r
pIα2trp32
5368 bp
trp p/o
RI
Ap^r
PstI

Fig 6

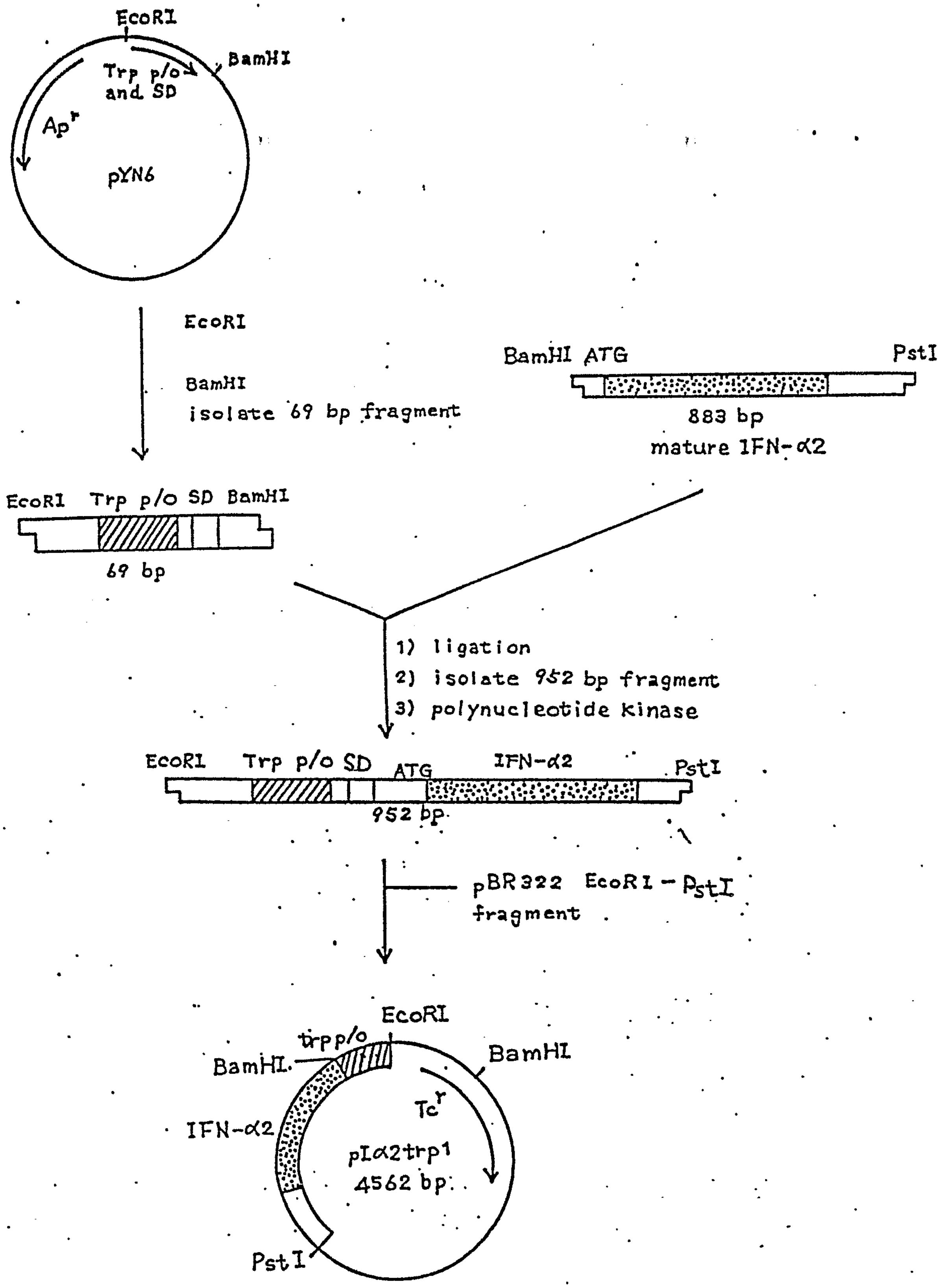
EcoRI
BamHI
Trp p/o and SD
Ap$^r$
pYN6
EcoRI
BamHI
isolate 69 bp fragment
BamHI ATG
PstI
883 bp
mature IFN-α2
EcoRI Trp p/o SD BamHI
69 bp
1) ligation
2) isolate 952 bp fragment
3) polynucleotide kinase
EcoRI Trp p/o SD ATG IFN-α2 PstI
952 bp
pBR322 EcoRI - PstI fragment
EcoRI
trp p/o
BamHI
BamHI
Tc$^r$
IFN-α2
pIα2trp1
4562 bp
PstI

Fig 7

```
    S1    S2          S23   1   2   3
    Met   Ala         Gly  Cys Asp Leu
    start
----ATGGCC------GCC TGT|GAT CTG-------
----TACCGG------CGG ACACTAG|AC-------
                          Sau3AI
```

↓ Sau3AI

↓ Synthetic DNA GATCCATGTGT
GTACACACTAG

```
      start  1   2   3
            Cys Asp Leu
|GATCCATGTGT|GATCTG-------
    |GTACACACTAG|AC-------
 BamHI          Sau3AI
```

Fig 8